# EUROPEAN PATENT APPLICATION

(11) **EP 4 283 296 A1**
(43) Date of publication of application: **29.11.2023**
(21) Application number: 21921464.0
(22) Date of filing: 06.12.2021
(51) Int. Cl.: G01N 33/569, C07K 14/45, C07K 14/005

(54) **VIRUS-LIKE PARTICLE COMPRISING TOXOPLASMA GONDII PROTEIN AND INFLUENZA VIRUS PROTEIN, AND DIAGNOSTIC METHOD USING SAME**

(30) Priority: 22.01.2021 KR 20210009692
(71) Applicant: Health Park Co., Ltd., Seoul 08826 (KR)
(72) Inventor: PARK, Hyunwoo, Seoul 04423 (KR); JANG, Yeong, Seoul 04423 (KR)
(74) Representative: Murgitroyd & Company
(86) International application number: PCT/KR2021/018383
(87) International publication number: WO 2022/158705

(57) **Abstract**

The present invention provides a composition for multiplex diagnosis of *Toxoplasma gondii* and/or influenza virus, comprising virus-like particles (VLPs) that comprise a *T. gondii* surface antigen protein, and VLPs that comprise an influenza virus M1 protein. In one embodiment, the VLPs comprise at least one protein from the group consisting of TgAMA1 VLP, TgROP4 VLP and TgROP18. The composition for multiplex diagnosis can react, with high sensitivity and specificity, to biological samples isolated from individuals infected with *T*. *gondii* and individuals infected with Influenza virus. Therefore, the composition for multiplex diagnosis can be used in a kit for multiplex diagnosis of toxoplasmosis and/or Influenza virus.

## Description

### [Technical field]

The present disclosure relates to a composition and a kit for diagnosing *Toxoplasma gondii* and/or influenza virus comprising virus-like particles (VLP) containing a *Toxoplasma gondii* protein and an influenza virus protein and a method for diagnosing *Toxoplasma gondii* and/or influenza virus infection using the same.

### [Background Art]

*Toxoplasma gondii* is an intracellular parasite that is the causative protozoa of toxoplasmosis in humans and animals. (Astrid M. Tenter et al). *Toxoplasma gondii* go through five development stages of oocyst, vegetative (tachyzoit), infectious (bradyzoit), blastomere (schizont), and reproductive mother (gametocyte). *Toxoplasma gondii* may be infected by ingesting water or vegetables contaminated by oocysts in cat feces, which is a definitive host, or by eating vegetables or eating *Toxoplasma gondii,* which presents as a cyst in meat, such as pigs, sheep, and cattle, which are intermediate hosts. It is estimated that more than one-third of the world's population is infected with *Toxoplasma gondii,* and it is reported that the infection rate in Korea is 2 to 25% depending on the group.

In particular, when *Toxoplasma gondii* infects the mother, the trophozoite may infect the fetus via the placenta. Infection with *Toxoplasma gondii* may lead to miscarriage or stillbirth in early fetuses, and congenital malformations such as visual impairment, hydrocephalus, and mental retardation in mid-late fetuses despite normal delivery. In addition, *Toxoplasma gondii,* which infects healthy individuals, may cause diseases such as lymphadenitis, chorioretinitis, and encephalomyelitis by destroying cells of the immune system and reticuloendothelial system, and when the host is immunocompromised, cysts proliferated in the brain are activated and may cause meningitis or chorioretinitis.

On the other hand, influenza is one of the main causes of human death due to respiratory viruses. Common symptoms include fever, sore throat, shortness of breath, and muscle pain, among others. Influenza viruses in the flu season are highly contagious, infecting 10% to 20% of the world's population and causing 250,000 to 500,000 deaths per year. What these diseases have in common is that the symptoms of other diseases have similar symptoms. Therefore, in order to reduce confusion with other diseases, various diagnostic methods, for example immunological methods such as a latex agglutination test, an antiserum diagnosis method (enzyme-linked immunosorbent assay, enzyme-linked immunospecific assay, ELISA), and DNA detection methods such as nested PCR and real-time PCR are used.

However, since the methods for diagnosing in the related are not fast enough to diagnose, or have matters of low economy or low sensitivity due to the use of and expensive device, a new diagnostic method with high speed, economy and sensitivity is required.

### [Related Art Document]

### [Non-Patent Document]

(Non-Patent Document 1) Astrid M. Tenter et al., "Toxoplasma gondii: from animals to humans", Int J Parasitol. 000 Nov; 30(12-13), 1217-1258

### [Disclosure]

### [Technical Problem]

Accordingly, the present inventors studied to develop a method with high speed and economy while being able to diagnose the *Toxoplasma gondii* and influenza virus with high sensitivity at the same time, and as a result, the present disclosure was completed by confirming that a diagnostic method and a diagnostic kit comprising the composition for diagnosing the VLP form according to the present disclosure have better immunological specificity and sensitivity than antigen lysate and an antigen protein.

### [Technical Solution]

In order to achieve the above purpose, the present disclosure provides a composition for multiplex diagnosis of toxoplasmosis and/or influenza virus, comprising virus-like particles comprising a *Toxoplasma gondii*-derived surface antigen protein, and influenza virus matrix protein 1 (M1).

In another aspect of the present disclosure, the present disclosure provides a kit for multiplex diagnosis of the Toxoplasmosis and/or influenza virus, comprising the composition for multiplex diagnosis of the toxoplasmosis and/or influenza virus.

Still another aspect of the present disclosure provides a method for providing information necessary for multiplex diagnosis of infection with *Toxoplasma gondii* and/or influenza virus, comprising: contacting a biological sample isolated from an individual with a composition for multiplex diagnosis of toxoplasmosis and/or influenza virus, comprising virus-like particles comprising a *Toxoplasma gondii*-derived surface antigen protein, and influenza virus matrix protein 1; and detecting antibodies that bind to virus-like particles.

### [Advantageous Effects]

The diagnostic composition comprising virus-like particles comprising *Toxoplasma gondii* antigen protein and influenza virus M1 protein according to the present disclosure may react with an autoantibody present in serum of a *Toxoplasma gondii*-infected individual with high specificity and sensitivity. In addition, the composition may react with autoantibodies present in an infected with influenza virus-infected individual. Accordingly, the virus-like particles may be usefully used for diagnosis of *Toxoplasma gondii* and/or influenza virus.

### [Description of Drawings]

FIGS. 1A to 1C shows a process of obtaining AMA1, ROP4 or ROP18 virus-like particles (VLPs) by transfecting rBV (recombinant baculovirus) comprising the AMA1, ROP4 or ROP18 genes and rBV comprising the influenza M1 gene into SF9 insect cells.
FIG. 2 shows a time-sequential view of an *in vivo* test procedure for obtaining infected serum from a mouse in one example embodiment of the present specification.
FIG. 3 shows a schematic diagram of a method for diagnosis performing ELISA using a VLP prepared in one example embodiment of the present specification.
FIG. 4 confirms the specificity of AMA1-M1-VLPs for infected serum by contacting serum from a naive mouse serum, a *P*. *berghei* ANKA (malaria protozoa) or *Toxoplasma gondii* (*T. gondii*) ME49-infected mouse with RH tachyzoite lysate antigen (TLA), AMA1-M1-VLPs, and AMA1 protein-coated immunoplates, respectively.
FIG. 5 shows the sensitivity of AMA1-M1-VLPs to infected serum by contacting the serum of a *T. gondii* ME49-infected mouse with TLA, AMA1-M1-VLPs, and AMA1 protein-coated immuneplates, respectively.
FIG. 6 shows the sensitivity of AMA1-M1-VLPs to infected serum by contacting the serum of a *T. gondii* ME49-infected human with TLA, AMA1-M1-VLPs, and AMA1 protein-coated immuneplates, respectively.
FIG. 7 confirms the specificity of ROP4-M1-VLPs for infected serum by contacting serum from a naive mouse serum, a *P. berghei* ANKA or *T. gondii* ME49-infected mouse with TLA, ROP4-M1-VLPs, and ROP4 protein-coated immunoplates, respectively.
FIG. 8 shows the sensitivity of ROP4-M1-VLPs to infected serum by contacting the serum of a *T. gondii* ME49-infected mouse with TLA, ROP4-M1-VLPs, and ROP4 protein-coated immuneplates, respectively.
FIG. 9 shows the sensitivity of ROP4-M1-VLPs to infected serum by contacting the serum of a *T. gondii* ME49-infected human with TLA, ROP4-M1-VLPs, and ROP4 protein-coated immuneplates, respectively.
FIG. 10 confirms the specificity of ROP18-M1-VLPs for infected serum by contacting serum from a naive mouse serum, a *P. berghei* ANKA or *T. gondii* ME49-infected mouse with TLA, ROP18-M1-VLPs, and ROP18 protein-coated immunoplates, respectively.
FIG. 11 shows the sensitivity of ROP18-M1-VLPs to infected serum by contacting the serum of a *T. gondii* ME49-infected mouse with TLA, ROP18-M1-VLPs, and ROP18 protein-coated immuneplates, respectively.
FIG. 12 shows the sensitivity of ROP18-M1-VLPs to infected serum by contacting the serum of a *T. gondii* ME49-infected human with TLA, ROP18-M1-VLPs, and ROP18 protein-coated immuneplates, respectively.
FIG. 13 shows the results of contacting naive mouse serum, serum of a *T*. *gondii* ME49-infected mouse, serum of a mouse inoculated with 10 µg of influenza M1 VLPs, and serum of a mouse inoculated with 10 µg of neuraminidase (NA) VLPs with immunoplates coated with TLA, AMA1-M1-VLPs, ROP4-M1-VLPs, and ROP18-M1-VLPs, respectively. As a result, it was confirmed that multiplex diagnosis of toxoplasmosis and influenza virus was possible using AMA1-M1-VLPs, ROP4-M1-VLPs, and ROP18-M1-VLPs.

### [Modes of the Invention]

One aspect of the present disclosure provides a composition for multiplex diagnosis of toxoplasmosis and/or influenza virus, comprising virus-like particles (VLPs) comprising *T gondii*-derived surface antigen protein, and influenza virus matrix protein 1 (M1).

The composition may be a composition for multiplex diagnosis of the toxoplasmosis and influenza virus at the same time.

As used herein, the term "virus-like particle" (VLP) or "virus-like particles" (VLPs) refers to non-infectious viral subunits with or without a viral protein. For example, the virus-like particle may completely lack a DNA or RNA genome. In one example embodiment, the virus-like particle may be prepared by a genetic engineering method, and includes influenza virus-derived matrix protein (M1) as a core protein. The virus-like particle of the present disclosure may be prepared by a genetic engineering method without a separate causative infectious agent, that is, *T. gondii.*

As used herein, the term "influenza virus matrix protein 1" (M1) is the core protein of influenza virus, and refers to a matrix protein that forms a coat inside a fat layer, which is the envelope of influenza virus. The influenza virus consists of subtypes designated A, B and C. The influenza virus is surrounded by a layer of a matrix protein (M1), which serves as a link between a core and a viral envelope, and the M1 protein may be widely used as the core protein in the development of influenza virus-like particles. The virus-like particles of the present specification includes influenza virus matrix protein 1 (M1) as a core protein, and in one example embodiment, M1 may have an amino acid sequence of SEQ ID NO: 1. In addition, M1 may be encoded by a nucleic acid sequence of SEQ ID NO: 5.

The *T. gondii*-derived surface antigen protein may be used in the present disclosure without being particularly limited to that type, as long as it is a protein capable of inducing immune activity against *T. gondii.*

Specifically, the surface antigen protein of the present specification may include various types of proteins such as SAG1(Membrane-associated surface antigen), SAG2, GRA1(secreted dense-granule protein), GRA2, GRA4, GRA7, MIC1(Microneme protein), MIC2, MIC4, MIC6, MIC7, MIC8, MIC9, MIC10, MIC 11, ROP1, ROP2, ROP3, ROP4, ROP5, ROP6, ROP7, ROP8, ROP9, ROP10, ROP11, ROP12, ROP13, ROP14, ROP15, ROP16, ROP17, ROP18, M2AP(MIC2 associated protein), AMA1(Plasmodium apical membrane antigen 1), and BAG1, derived from *T. gondii,* and may preferably comprise at least one of AMA1, ROP4 and ROP18.

In this case, AMA1 may have an amino acid sequence of SEQ ID NO: 2. In addition, ROP4 may have an amino acid sequence of SEQ ID NO: 3. In addition, ROP18 may have an amino acid sequence of SEQ ID NO: 4.

In addition, AMA1 may be encoded by a nucleic acid sequence of SEQ ID NO: 6, ROP4 may be encoded by a nucleic acid sequence of SEQ ID NO: 7, and ROP18 may be encoded by a nucleic acid sequence of SEQ ID NO: 8.

Here, since the antigenic protein introduced on the surface of the virus-like particle has higher antigenicity than a purely isolated recombinant protein, the protein may form an effective neutralizing antibody, and thus may be used as a composition for multiplex diagnosis of toxoplasmosis and influenza virus.

A ratio of *T. gondii-*derived surface antigen protein and influenza virus matrix protein 1 in the virus-like particles may be present in a ratio of 5:1 to 1:5. The ratio may also be present in a ratio of 3:1, 1:1 or 1:3. Preferably, the ratio may be 3:1.

The virus-like particles may be prepared using a vector comprising a gene encoding the *T. gondii*-derived surface antigen protein and a vector comprising a gene encoding the influenza virus matrix protein 1. Specifically, the virus-like particles may be prepared by coinfecting host cells with a recombinant baculovirus comprising a gene encoding the *T. gondii-*derived surface antigen protein and a recombinant baculovirus comprising a gene encoding the influenza virus matrix protein 1.

In this case, insect cells may be used as the host cells. The insect cells may be any cell developed or commercially available as a host system for gene expression, and may include one or more selected from the group consisting of *Spodoptera frugiperda* SF21, SF9, *Trichoplusia ni*, *Anticarsa gemmitalis*, *Bombyx mori*, *Estigmene acrea*, *Heliothis virescens*, *Leucania separata*, *Lymantria dispar*, *Malacasoma disstria*, *Mammestra brassicae*, *Manduca sexta*, *Plutella zylostella*, *Spodoptera exigua*, and *Spodoptera littorlis*, but are not limited thereto. In one example, SF9 cell was used.

In addition, during co-infection, the virus-like particles may be prepared by infecting host cells with a recombinant baculovirus comprising a gene encoding the *T. gondii*-derived surface antigen protein and a recombinant baculovirus comprising a gene encoding the influenza virus matrix protein 1 at a ratio of 3:1.

In particular, the virus-like particle comprising the *T. gondii*-derived surface antigen protein and the influenza virus matrix protein 1 has very good specificity and sensitivity. Thus, it may be used to detect autoantibodies produced in a *T. gondii* and/or influenza virus-infected individual.

For example, the VLPs may be used to determine the presence or absence of an anti-AMA1 antibody, an anti-ROP4 antibody, or an anti-ROP18 antibody produced in an individual upon infection with *T. gondii.* In addition, it may be used to determine the presence or absence of anti-M1 antibodies produced in an individual upon infection with influenza virus.

In a specific experimental examples of the present disclosure, it was confirmed that ELISA was performed using sera obtained from *T. gondii*-infected mice and human patients and the VLPs, and exhibited high sensitivity. it was confirmed that since the VLPs did not show a significant antibody response to the serum of malaria protozoa (P. berghei ANKA)-infected mouse or the serum of a naive mouse, the VLPs may be used to diagnose a *T. gondii-*infected individual. In addition, even though the same ELISA experiment was performed, it was confirmed that since almost no antibody response was observed compared to the VLPs for proteins such as RH TLA (tachyzoite lysate antigen) or AMA1, ROP4, and ROP18, even serum of the *T. gondii* infected-derived mouse, and the VLPs of the present disclosure are more effective in sensitively diagnosing *T. gondii* infection compared to the case of using a *T. gondii-*derived protein or a lysate of *T. gondii.*

Furthermore, the VLPs may more efficiently diagnose the infection of *T. gondii* and influenza virus in individuals infected with *T. gondii* and influenza virus at the same time.

In a specific experimental example of the present disclosure, it was confirmed that the VLPs of the present disclosure exhibited a significant antigen-antibody reaction not only against the serum of the *T*. *gondii*-infected mouse, but also against the serum of the influenza virus-infected mouse, and thus the VLPs may be usefully used for multiplex diagnosis of the *T. gondii* and influenza virus at the same time.

Another aspect of the present disclosure provides provides a kit for multiplex diagnosis of the Toxoplasmosis and/or influenza virus, comprising the composition for diagnosis of the toxoplasmosis and/or influenza virus. The diagnostic kit may comprise the virus-like particle comprising the *T. gondii*-derived surface antigen protein and the influenza virus matrix protein 1 (M1).

In addition, the diagnostic kit may be a diagnostic kit for multiplex diagnosis of the toxoplasmosis and influenza virus at the same time.

In this case, the composition for diagnosing toxoplasmosis and/or influenza virus, a toxoplasmosis-derived surface antigen protein, an influenza virus matrix protein, and virus-like particles are as described above. In one example embodiment, the kit may be manufactured using AMA1-M1-VLPs, which are VLPs expressing a *T. gondii* AMA1 protein, ROP4-M1-VLPs, which are VLPs expressing a *T*. *gondii* ROP4 protein, and ROP18-M1-VLPs, which are VLPs expressing a *T*. *gondii* ROP18 protein. In this case, the VLPs may be present in a detection portion of the diagnostic kit.

As used herein, the term "detection portion" refers to a portion including a support on which a VLP composition comprising a *T. gondii* surface antigen protein is immobilized, and means a site where at least one of an antibody to the *T. gondii* and an antibody to the influenza virus, and the VLP composition react specifically.

The composition for multiplex diagnosis of toxoplasmosis and/or influenza virus of the detection portion is coated on a support (for example, nitrocellulose membrane) of the detection portion using a dispensing device, and diagnosis of *T. gondii* or multiplex diagnosis of *T. gondii* and influenza virus may be performed by contacting the corresponding portion with a biological sample isolated from the individual.

The diagnostic kit may be at least one selected from the group consisting of an enzyme-linked immunosorbent assay (ELISA) kit, a radioimmnoassay (RIA) kit, a Sandwich assay kit, a Western Blot kit, an immunoblot assay kit, and an immunohistochemical staining kit, but is not limited thereto.

In one example embodiment, The diagnostic kit may individually or simultaneously diagnose *T. gondii* and influenza virus infection by detecting the presence or absence of an IgG antibody, IgA or IgM antibodies in a biological sample isolated from the individual, and in the process, at least one selected from the group consisting of enzyme-linked immunosorbent assay (ELISA), radioimmnoassay (RIA), sandwich assay, Western Blot, immunoblot assay, and Immunohistochemical staining may be performed including , but is not limited thereto.

Another aspect of the present disclosure provides a method for providing information necessary for multiplex diagnosis of *T. gondii* and/or influenza virus infection, comprising: contacting a biological sample isolated from an individual with a composition for multiplex diagnosis of toxoplasmosis and/or influenza virus comprising virus-like particles comprising a *T. gondii*-derived surface antigen protein and influenza virus matrix protein 1; and detecting antibodies that bind to the virus-like particle.

In this case, the composition for diagnosing toxoplasmosis and/or influenza virus, a toxoplasmosis-derived surface antigen protein, an influenza virus matrix protein, and virus-like particles are as described above. In addition, the term "biological sample isolated from an individual" as used herein is preferably a body fluid secreted outside the body, includes blood, serum, plasma, urine, tears, saliva, milk, and the like, and more preferably may be blood obtained from an individual, but is not limited thereto. In this case, the individual may be a mammal such as a dog, a cat, or a human, and preferably a human.

Detection of an antibody that binds to the virus-like particle may include at least one selected from the group consisting of enzyme-linked immunosorbent assay (ELISA), radioimmnoassay (RIA), sandwich assay, Western blot, immunoblot assay, and immunohistochemical staining.

A method of detecting an antibody that binds to the virus-like particle may be a method of detecting an antigen-antibody reactant or an analyte. Methods known to those skilled in the art may be used depending on the type of the antigen-antibody reactant or the analyte, and specifically, may use a naked eye, a detection device, a detection reagent, and the like. A confocal laser scanner may be used as the detection device, and the detection reagent may include a predetermined labeled reagent, an auxiliary specific binding member, and/or a component of a signaling system that enable external identification of the presence of the analyte to be tested through the naked eye or other devices. Labeled detection reagents are already well known to those skilled in the art.

Examples of such labels include catalysts, enzymes (for example, phosphatase, peroxidase, and the like, more specific examples being basic phosphatase and horseradish peroxidase used in combination with an enzyme substrate), enzyme substrates (for example, nitroblue tetrazolium, 3,5',5,5'-tetranitrobenzidine, 4-methoxy-1-naphthol, 4-chloro-1-naphthol, 5-bromo-4-chloro-3-indolyl phosphate, dioxetane as a chemiluminescent enzyme substrate, and derivatives and analogs thereof), fluorescent compounds (for example, fluorescein, phycobiliprotein, rhodamine, and derivatives and analogs thereof), chemiluminescent compounds, non-metal sol such as metal sol, dye sol, particulate latex, color indicator, color matter included in liposome, carbon sol, and selenium, but are not limited thereto.

Another aspect of the present disclosure provides a method for multiple diagnosis of the *T. gondii* and/or influenza virus infection, comprising: contacting a biological sample isolated from an individual with a composition for diagnosing toxoplasmosis and/or influenza virus; and detecting antibodies that bind to virus-like particles.

Another aspect of the present disclosure provides diagnostic use of toxoplasmosis and/or influenza virus infection of virus-like particles comprising *T. gondii*-derived surface antigen protein; and influenza virus matrix protein 1 (M1). Specifically, the use may be for diagnosing toxoplasmosis or multiplex diagnosis of toxoplasmosis and influenza virus at the same time.

Hereinafter, the present disclosure will be described in more detail through examples. These examples are only for illustrating the present disclosure, and it will be apparent to those of ordinary skill in the art that the scope of the present disclosure is not to be construed as being limited by these examples.

### I. Preparation of VLP comprising Toxoplasma gondii antigen and influenza antigen

### Preparation Example 1. Preparation of baculovirus expressing Toxoplasma gondii antigen

### Preparation Example 1.1. Obtaining Toxoplasma gondii cDNA

To extract RNA encoding AMA1, ROP4, and ROP18 from *Toxoplasma gondii* RH, RNA was obtained from *Toxoplasma gondii* RH samples. Thereafter, the extracted antigen RNA was subjected to RT-PCR to obtain *Toxoplasma gondii-derived* cDNA. Then, dsDNA was obtained based on the obtained cDNA.

### Preparation Example 1.2. Obtaining antigen cDNA

Primers were designed and ordered to amplify AMA1, ROP4, and ROP18, for the purpose of performing PCR on the target gene. Distilled water was added to a mixture of 0.25 µl of PCR enzyme TaKaRa Ex Taq^{™}, 5 µl of 10X Ex Taq buffer, 4 µl of a dNTP mixture, 1 µl of template, 2 µl of forward primer, and 2 µl of reverse primer to make a total volume of 50 µl. Thereafter, PCR was performed for 30 to 40 cycles at 98°C for 10 seconds, 55°C for 30 seconds, and 72°C for 1 minute. Thereafter, a PCR amplification product diluted with a DNA marker and a loading dye (6x) was loaded on an agarose gel. Thereafter, the PCR amplification product was confirmed using UV light and *etc.* Thereafter, DNA encoding an antigen was extracted from the gel where a desired gene was positioned.

In the above, for a gene of AMA1, 5'-AAAGAATTCACCATGGGGCTCGTGGGCGTACA-3' (SEQ ID NO: 11) was used as a forward primer and 5'-TTACTCGAGCTAGTAATCCCCCTCGACCA-3' (SEQ ID NO: 12) was used as a reverse primer, for a gene of ROP4, 5'-AAAGCATGCACCATGGGGCACCCTACCTCTTT-3' (SEQ ID NO: 13) was used as a forward primer and 5'-TTAGGTACCTCACGTTTCCGGTGGTGGCAT-3' (SEQ ID NO: 14) was used as a reverse primer, and for a gene of ROP18 gene, 5'-AAAGGATCCACCATGTTTTCGGTACAGCGGCC-3' (SEQ ID NO: 15) was used as a forward primer and 5'-TTATCTAGATTATTCTGTGTGGAGATGTTC-3' (SEQ ID NO: 16) was used as a reverse primer.

### Preparation Example 1.3. Amplifying antigen gene

DNAs of AMA1, ROP4, and ROP18, which are *Toxoplasma gondii* antigens extracted in Preparation Example 1.2, were loaded onto a TA vector. Thereafter, the TA vector loaded with the antigen gene was transformed into E. coli cells and cultured. After culturing the transformed E. coli cells, genes encoding AMA1, ROP4, and ROP18 were obtained therefrom.

### Preparation Example 1.4. Preparation of pFast-Bac plasmid loaded with antigen gene

The AMA1, ROP4, and ROP18 antigen genes obtained in Preparation Example 1.3 were loaded onto a pFast-Bac vector, respectively. The pFast-Bac vector loaded with the antigen gene was transformed into DH10B cells. The transformed cells were cultured to obtain a large amount of the pFast-Bac vector loaded with the antigen gene.

### Preparation Example 1.5. Preparation of baculovirus expressing Toxoplasma gondii antigen

A pFast-Bac vector loaded with AMA1, ROP4, and ROP18 DNA were transfected into SF9 cells using cellfectin II (Invitrogen, USA). Recombinant baculovirus (rBV) expressing AMA1, ROP4, and ROP18 was prepared using a Bac-to-Bac expression system (Invitrogen) according to a manufacturer's manual.

In addition, rBV expressing influenza virus M1 was prepared in the same manner as above. At this time, a gene of M1 was amplified by RT-PCR using 5'-AAAGGATCCACCATGAGTCTTCTAACCGAGGT-3' (SEQ ID NO: 9) as a forward primer and 5'-TTACTCGAGTTACTCTAGCTCTATGTTGAC-3' (SEQ ID NO: 10) as a reverse primer.

### Example 1. Preparation of VLP comprising Toxoplasma gondii antigen and influenza antigen: AMA1-M1-VLP

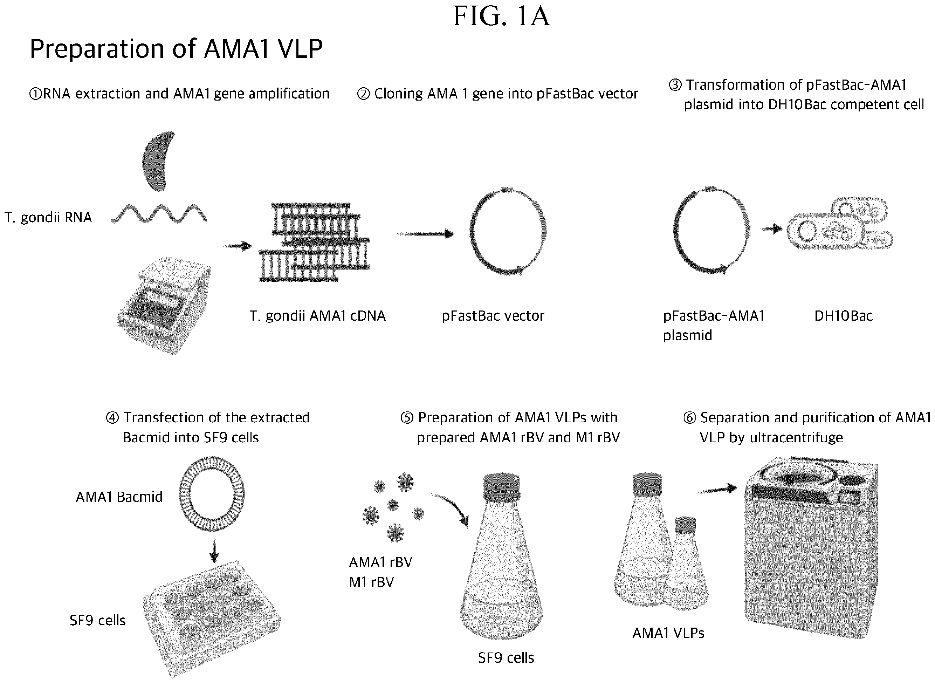

To prepare a first *Toxoplasma gondii* VLP comprising AMA1 and M1 proteins, SF9 cells were co-infected with AMA1 rBV and M1 rBV, prepared in Preparation Example 1. At this time, the SF9 cells were infected with M1 rBV and AMA1 antigen rBV in a ratio of 1:3. Thereafter, the infected SF9 cells were cultured, and harvesting was performed when a death rate of the SF9 cell reached 30% to 40%. Specifically, the cultured cells and culture solution were centrifuged at 6,000 rpm for 30 minutes to collect a supernatant, and then pelleted by centrifugation at 30,000 rpm and 4°C for 30 minutes. VLPs were purified via a sucrose gradient. In addition, VLPs were re-suspended in phosphate-buffered saline (PBS) until use. VLPs expressing the AMA1 antigen and the M1 antigen were named AMA1-M1-VLPs. An example of a process for preparing the AMA1-M1-VLP is shown in FIG. 1a.

### Example 2. Preparation of VLPs comprising Toxoplasma gondii antigen and influenza antigen: ROP4-M1-VLPs

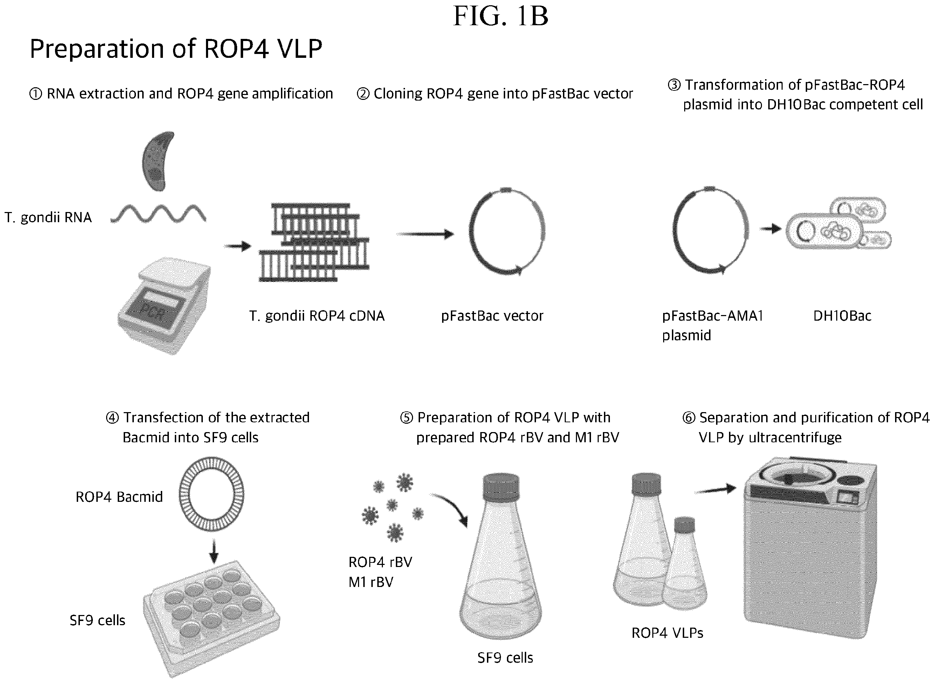

In addition, in order to prepare a second *Toxoplasma gondii* VLP comprising ROP4 and M1 proteins, SF9 were co-infected with ROP4 rBV and M1 rBV, prepared in Preparation Example 1, in the same manner as in Example 1 above to obtain ROP4-M1-VLPs (FIG. 1B).

### Example 3. Preparation of VLPs comprising Toxoplasma gondii antigen and influenza antigen: ROP18-M1-VLPs

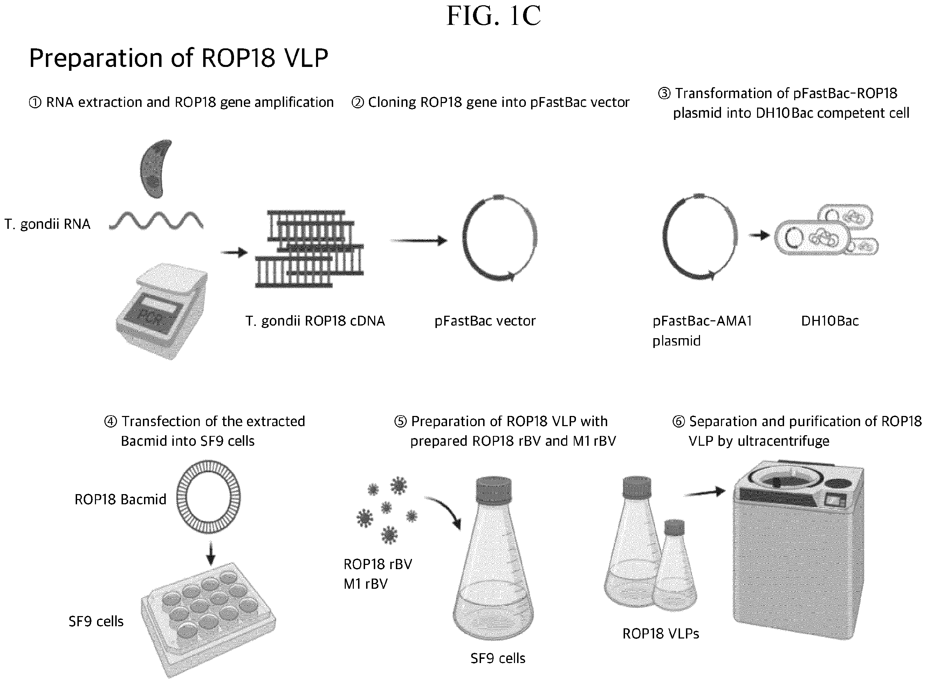

In addition, in order to prepare a third *Toxoplasma gondii* VLP comprising ROP18 and M1 proteins, SF9 were co-infected with ROP18 rBV and M1 rBV, prepared in Preparation Example 1, in the same manner as in Example 1 above to obtain ROP18-M1-VLPs (FIG. 1C).

### II. Diagnosis of Toxoplasma gondii and influenza using VLPs

### Preparation Example 2. Collection of serum from mice infected with Toxoplasma gondii and malaria

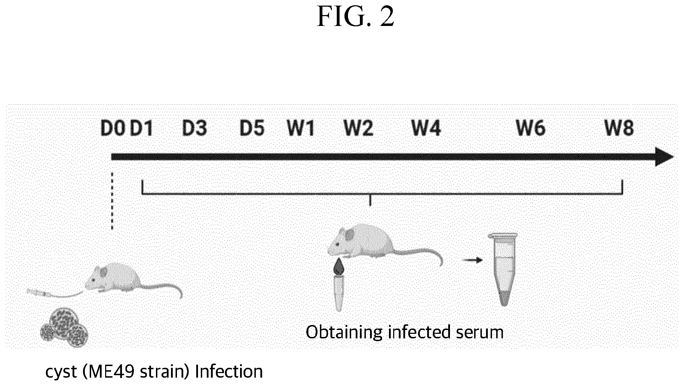

7-week-old female BALB/c mice were orally infected with cysts of *Toxoplasma gondii* (ME49 strain) at 300 cysts/mouse (hd), and infectious serum was obtained by centrifugation after blood collection from orbital venous plexus. An exemplary order for an *in-vivo* process is shown in FIG. 2. In addition, after infecting a mouse with 0.5% (0.5 × 10⁴) of malaria protozoa (*P. berghei* ANKA), serum was collected from the infected mice.

### Experimental example 1. AMA1-M1-VLPs and IgG antibody reaction test

In order to confirm whether the AMA1-M1-VLPs of the present disclosure, obtained through Example 1, may actually diagnose a *Toxoplasma gondii*-infected individual, it was confirmed by ELISA whether AMA1-M1-VLPs react with *Toxoplasma gondii-specific* antibodies in serum, using serum from *Toxoplasma gondii-infected* mice and human patients (Experimental Examples 1.1 to 1.3).

### Experimental Example 1.1. Specificity test of AMA1-M1-VLPs and IgG antibody response in mouse serum

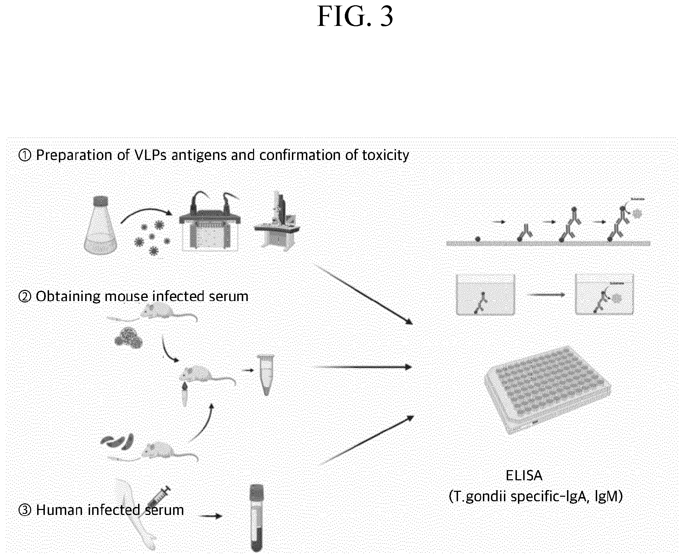

AMA1-M1-VLPs, AMA1 protein, and TLA (RH Tachyzoite lysate antigen) obtained in Example 1 were coated on a 96-well immunoplate at a concentration of 4 µg/ml, respectively. The TLA refers to a lysate antigen of a tachyzoite state during the life cycle of *Toxoplasma gondii* RH. Thereafter, ELISA was performed by diluting 14 mouse serum infected with malaria protozoa (*P. berghei* ANKA) and 14 mouse serum infected with *T. gondii* ME49 300 cysts by 100-fold (FIG. 3). Serum of a mouse not infected with malaria protozoa or *toxoplasma gondii* was used as a control (Naive).

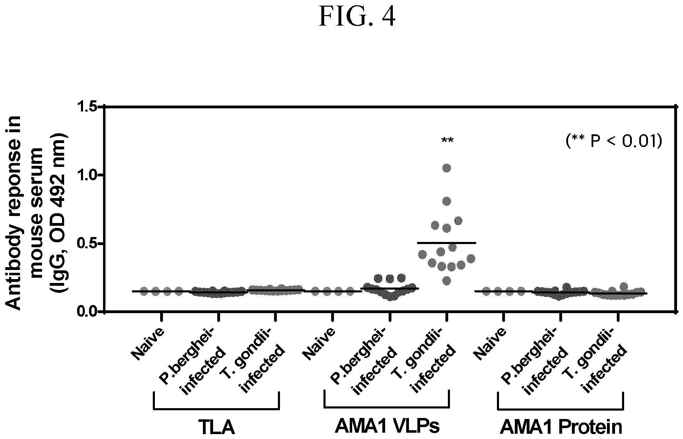

As a result, among three coated antigens, TLA and AMA1 protein antigens did not react with any serum. In contrast, AMA1-M1-VLPs hardly reacted with a naive serum and a serum infected with the malaria protozoa, and showed high reactivity only to serum infected with *Toxoplasma gondii,* confirming the specificity between AMA1-M1-VLPs and *Toxoplasma gondii*-specific IgG antibody in serum (FIG. 4).

### Experimental Example 1.2. Sensitivity test of AMA1-M1-VLPs and IgG antibody response in mouse serum

In addition to confirming that AMA-M1-VLPs specifically bind to a *Toxoplasma gondii*-specific IgG antibody in Experimental Example 1.1, in order to confirm the sensitivity of these reactions, serum comprising the IgG antibody was treated at various concentrations to perform the same experiment as Example 1.1. AMA1-M1-VLPs, an AMA1 protein, and TLA (RH Tachyzoite lysate antigen) obtained in Example 1 were coated on a 96-well immunoplate at a concentration of 4 µg/ml, respectively. Thereafter, ELISA was performed by diluting 14 mouse serum infected with 300 cysts of *T. gondii* ME49 by 100-fold, 300-fold, and 900-fold, respectively (FIG. 3). Serum of a mouse not infected with *toxoplasma gondii* was used as a control (Naive).

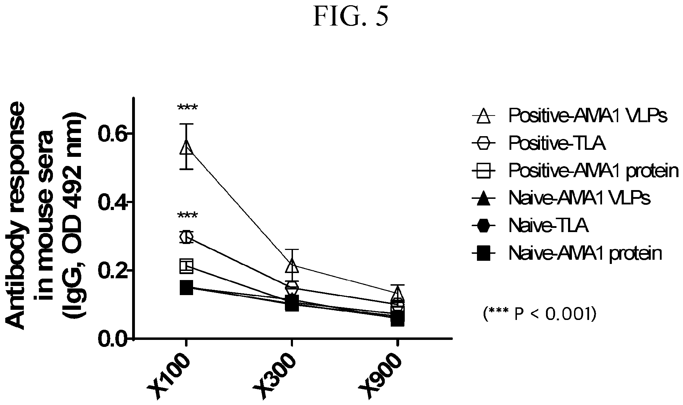

As a result, as with Experimental Example 1.1, AMA1-M1-VLPs were found to have the highest specificity for mouse serum comprising *Toxoplasma gondii-specific* IgG, and among them, the highest sensitivity for 100-fold diluted serum concentration (FIG. 5).

### Experimental Example 1.3. Sensitivity test of AMA1-M1-VLPs and IgG antibody response in human serum

In order to confirm the sensitivity of an antibody response to the serum of a *Toxoplasma gondii*-infected human, experiments were performed in a similar manner to Experimental Examples 1.1 and 1.2. AMA1-M1-VLPs, AMA1 protein, and TLA (RH Tachyzoite lysate antigen) obtained in Example 1 were coated on a 96-well immunoplate at a concentration of 4 µg/ml, respectively. Thereafter, ELISA was performed by diluting 10 serum of *T. gondii*-infected patients by 100-fold, 300-fold, and 900-fold, respectively (FIG. 3). Serum of a mouse not infected with *toxoplasma gondii* was used as a control (Naive).

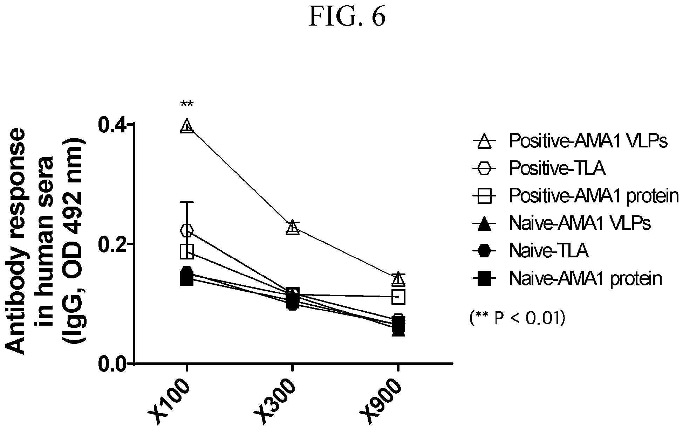

As a result, as with an experiment using mouse serum, AMA1-M1-VLPs were found to have the highest specificity for human serum comprising *Toxoplasma gondii-specific* IgG, and among them, the highest sensitivity for 100-fold diluted serum concentration (FIG. 6).

### Experimental example 2. ROP4-M1-VLPs and IgG antibody reaction test

As with Experimental Example 1, in order to confirm whether the ROP4-M1-VLPs of the present disclosure obtained in Example 2 may actually diagnose an individual infected with *Toxoplasma gondii,* ELISA was performed to confirm whether ROP4-M1-VLPs react with *Toxoplasma gondii-specific* antibodies in serum using serum from *Toxoplasma gondii-infected* mice and human patients (Experimental Examples 2.1 to 2.3).

### Experimental Example 2.1. Specificity test of ROP4-M1-VLPs and IgG antibody response in mouse serum

ROP4-M1-VLPs, ROP4 protein, and TLA (RH Tachyzoite lysate antigen) obtained in Example 2 were coated on a 96-well immunoplate at a concentration of 4 µg/ml, respectively. Thereafter, ELISA was performed by diluting 14 mouse serum infected with malaria protozoa(*P*. *berghei* ANKA) and 14 mouse serum infected with *T. gondii* ME49 300 cysts by 100-fold (FIG. 3). Serum of a mouse not infected with malaria protozoa or *toxoplasma gondii* was used as a control (Naive).

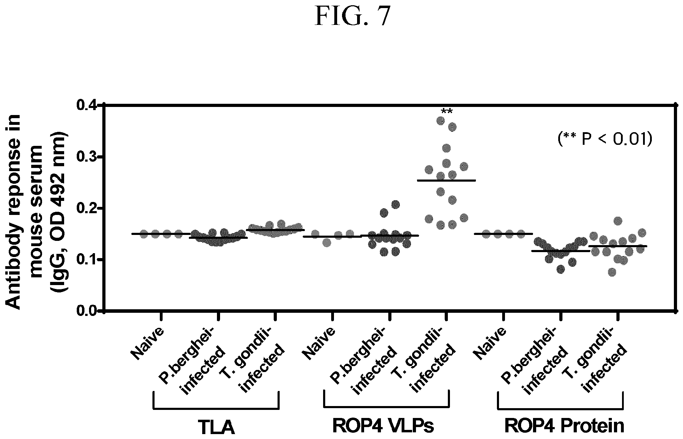

As a result, among three coated antigens, TLA and AMA1 protein antigens did not react with any serum. In contrast, ROP4-M1-VLPs hardly reacted with an Naive serum and a serum infected with the malaria protozoa, and showed high reactivity only to serum infected with *Toxoplasma gondii,* confirming the specificity between ROP4-M1-VLPs and *Toxoplasma gondii*-specific IgG antibody in serum (FIG. 7).

### Experimental Example 2.2. Sensitivity test of ROP4-M1-VLPs and IgG antibody response in mouse serum

In addition to confirming that ROP4-M1-VLPs specifically bind to a *Toxoplasma gondii*-specific IgG antibody in Experimental Example 2.1, in order to confirm the sensitivity of these reactions, serum comprising the IgG antibody was treated at various concentrations to perform the same experiment as Example 2.1. ROP4-M1-VLPs, ROP4 protein, and TLA (RH Tachyzoite lysate antigen) obtained in Example 2 were coated on a 96-well immunoplate at a concentration of 4 µg/ml, respectively. Thereafter, ELISA was performed by diluting 14 mouse serum infected with 300 cysts of *T. gondii* ME49 by 100-fold, 300-fold, and 900-fold, respectively (FIG. 3). Serum of a mouse not infected with *Toxoplasma gondii* was used as a control (Naive).

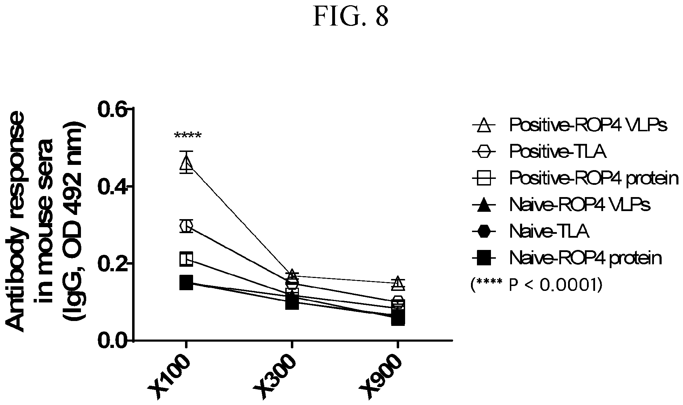

As a result, as with Experimental Example 2.1, ROP4-M1-VLPs were found to have the highest specificity for mouse serum comprising *Toxoplasma gondii*-specific IgG, and among them, the highest sensitivity for 100-fold diluted concentration (FIG. 8).

### Experimental Example 2.3. Sensitivity test of ROP4-M1-VLPs and IgG antibody response in human serum

In order to confirm the sensitivity of an antibody response to the serum of a *Toxoplasma gondii*-infected human, experiments were performed in a similar manner to Experimental Examples 2.1 and 2.2. ROP4-M1-VLPs, ROP4 protein, and TLA (RH Tachyzoite lysate antigen) obtained in Example 2 were coated on a 96-well immunoplate at a concentration of 4 µg/ml, respectively. Thereafter, ELISA was performed by diluting 10 serum of *T. gondii*-infected patients by 100-fold, 300-fold, and 900-fold, respectively (FIG. 3). Serum of a mouse not infected with *Toxoplasma gondii* was used as a control (Naive).

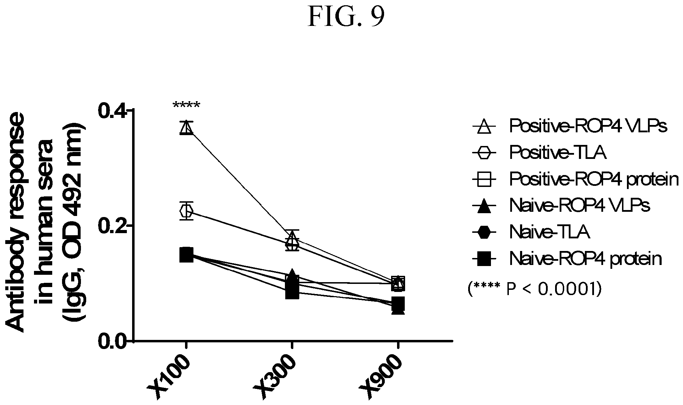

As a result, as with an experiment using mouse serum, AMA1-M1-VLPs were found to have the highest specificity for human serum comprising *Toxoplasma gondii*-specific IgG, and among them, the highest sensitivity for 100-fold diluted serum concentration (FIG. 9).

### Experimental example 3. ROP18-M1-VLPs and IgG antibody reaction test

As with Experimental Example 1, in order to confirm whether the ROP18-M1-VLPs of the present disclosure obtained in Example 3 may actually diagnose an individual infected with *Toxoplasma gondii*-infected individual, ELISA was performed to confirm whether ROP18-M1-VLPs react with *Toxoplasma gondii*-specific antibodies in serum using serum from *Toxoplasma gondii*-infected mice and human patients (Experimental Examples 3.1 to 3.3).

### Experimental Example 3.1. Specificity test of ROP18-M1-VLPs and IgG antibody response in mouse serum

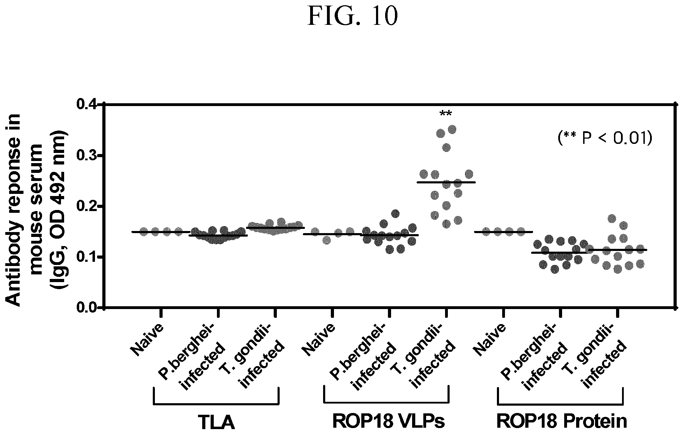

ROP18-M1-VLPs, ROP18 protein, and TLA (RH Tachyzoite lysate antigen) obtained in Example 3 were coated on a 96-well immunoplate at a concentration of 4 µg/ml, respectively. Thereafter, ELISA was performed by diluting 14 mouse sera infected with malaria protozoa (*P. berghei* ANKA) and 14 mouse sera infected with *T. gondii* ME49 300 cysts by 100-fold (FIG. 3). Serum of a mouse not infected with malaria protozoa or Toxoplasma gondii was used as a control (Naive). As a result, among three coated antigens, TLA and AMA1 protein antigens did not react with any serum. In contrast, ROP18-M1-VLPs hardly reacted with a naive serum and a serum infected with the malaria protozoa, and showed high reactivity only to serum infected with *Toxoplasma gondii,* confirming the specificity between ROP18-M1-VLPs and *Toxoplasma gondii*-specific IgG antibody in serum (FIG. 10).

### Experimental Example 3.2. Sensitivity test of ROP18-M1-VLPs and IgG antibody response in mouse serum

In addition to confirming that ROP18-M1-VLPs specifically bind to a *Toxoplasma gondii*-specific IgG antibody in Experimental Example 3.1, in order to confirm the sensitivity of these reactions, serum comprising the IgG antibody was treated at various concentrations to perform the same experiment as Example 3.1. ROP18-M1-VLPs, ROP18 protein, and TLA (RH Tachyzoite lysate antigen) obtained in Example 3 were coated on a 96-well immunoplate at a concentration of 4 µg/ml, respectively. Thereafter, ELISA was performed by diluting 14 mouse serum infected with 300 cysts of *T. gondii* ME49 by 100-fold, 300-fold, and 900-fold, respectively (FIG. 3). Serum of a mouse not infected with *Toxoplasma gondii* was used as a control (Naive).

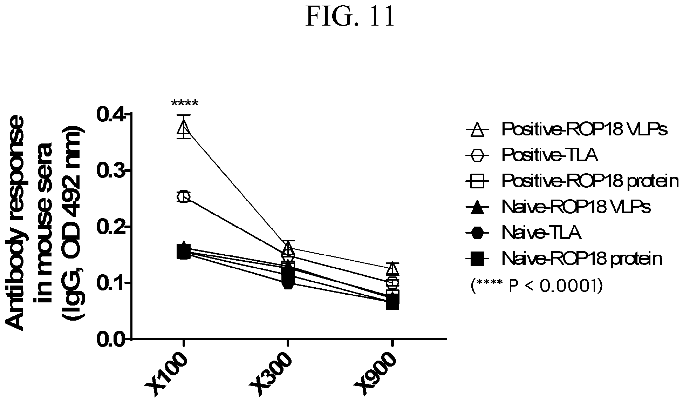

As a result, as with Experimental Example 3.1, ROP18-M1-VLPs were found to have the highest specificity for mouse serum comprising *Toxoplasma gondii*-specific IgG, and among them, the highest sensitivity for 100-fold diluted serum concentration (FIG. 11).

### Experimental Example 3.3. Sensitivity test of ROP18-M1-VLPs and IgG antibody response in human serum

In order to confirm the sensitivity of an antibody response to the serum of a *Toxoplasma gondii*-infected human, experiments were performed in a similar manner to Experimental Examples 3.1 and 3.2. ROP18-M1-VLPs, ROP18 protein, and TLA (RH Tachyzoite lysate antigen) obtained in Example 3 were coated on a 96-well immunoplate at a concentration of 4 µg/ml, respectively. Thereafter, ELISA was performed by diluting 10 sera of *T. gondii*-infected patients by 100-fold, 300-fold, and 900-fold, respectively (FIG. 3). Serum of a mouse not infected with *Toxoplasma gondii* was used as a control (Naive).

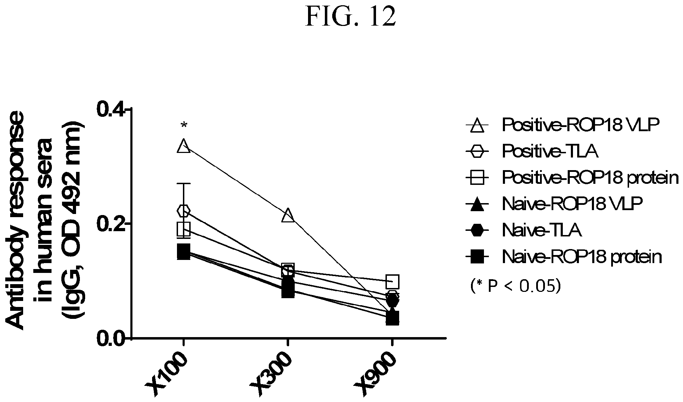

As a result, as with an experiment using mouse serum, AMA1-M1-VLPs were found to have the highest specificity for human serum comprising *Toxoplasma gondii-*specific IgG, and among them, the highest sensitivity for 100-fold diluted serum concentration (FIG. 12).

### Experimental example 4. Simultaneous diagnosis of Toxoplasma gondii and influenza using AMA1-M1-VLPs, ROP4-M1-VLPs, and ROP18-M1-VLPs

AMA1-M1-VLPs, ROP4-M1-VLPs, ROP18-M1-VLPs and TLA (RH Tachyzoite lysate antigen) obtained in Examples 1 to 3 were coated on a 96-well immunoplate at a concentration of 4 µg/ml, respectively.

Thereafter, naive mouse serum, serum of mouse infected with *T. gondii* ME49 300 cysts, serum of mouse inoculated with 10 µg of influenza M1 VLPs, and serum of mouse inoculated with 10 µg of neuraminidase (NA) VLPs were diluted 100-fold for performing ELISA.

Here, NA VLPs were prepared using the same method as the preparation of *T. gondii* VLPs by amplifying cDNA of NA from influenza virus A/PR/8/34 (H1N1).

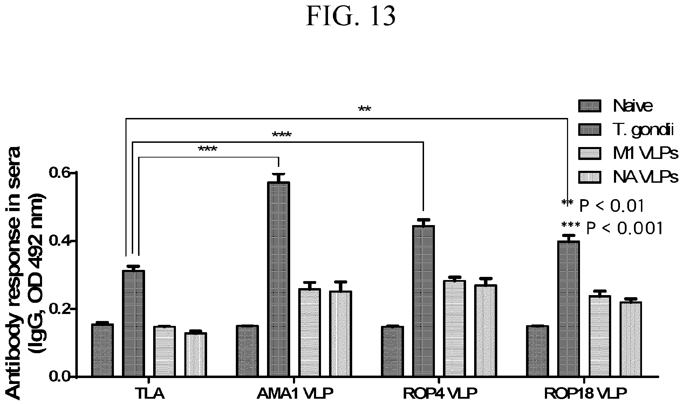

As a result, when OD values were measured at 492 nm on AMA1-M1-VLPs, ROP4-M1-VLPs, and ROP18-M1-VLPs-coated immunoplates among the above four antigen-coated immunoplates, a significantly high IgG antibody response was shown in the serum of mouse infected with *Toxoplasma gondii.* In addition, by measuring a value of 0.2 or more for the serum of a mouse inoculated with 10 µg of influenza M1 VLPs or NA VLPs at the same time, it was confirmed that multiplex diagnosis of toxoplasmosis and influenza virus was possible using the AMA1-M1-VLPs, ROP4-M1-VLPs and ROP18-M1-VLPs-coated immunoplates (FIG. 13).

In conclusion, it was confirmed that an anti-AMA1 antibody, an anti-ROP4 antibody, and/or an anti-ROP18 antibody present in the serum of a *Toxoplasma gondii*-infected individual can be detected with high sensitivity and specificity, using AMA1-M1-VLPs, ROP4-M1-VLPs, and ROP18-M1-VLPs. In addition, it was confirmed that an anti-M1 antibody present in the serum of an influenza virus-infected individual can also be detected with high sensitivity and specificity, using AMA1-M1-VLPs, ROP4-M1-VLPs, and ROP18-M1-VLPs. Therefore, the AMA1-M1-VLPs, ROP4-M1-VLPs, and POP 18-M1-VLPs of the present application can be used as a composition for simultaneously diagnosing an individual infected with *Toxoplasma gondii* and/or an individual infected with influenza virus.

## Claims

1. A composition for multiplex diagnosis of toxoplasmosis and/or influenza virus, comprising virus-like particles, wherein the virus-like particles comprise *Toxoplasma gondii-*derived surface antigen protein, and influenza virus matrix protein 1 (M1).

2. The composition of claim 1, wherein the *Toxoplasma gondii*-derived surface antigen protein is at least one protein selected from the group consisting of AMA1, ROP4, and ROP18.

3. The composition of claim 1, wherein M1 has an amino acid sequence of SEQ ID NO: 1.

4. The composition of claim 2, wherein
the AMA1 has an amino acid sequence of SEQ ID NO: 2,
the ROP4 has an amino acid sequence of SEQ ID NO: 3, and
the ROP18 has an amino acid sequence of SEQ ID NO: 4.

5. The composition of claim 1, wherein a ratio of the *Toxoplasma gondii-*derived surface antigen protein to the influenza virus matrix protein 1 in the virus-like particles is 3:1.

6. The composition of claim 1, wherein the virus-like particles are prepared by coinfecting a host cell with a recombinant baculovirus comprising a gene encoding the *Toxoplasma gondii*-derived surface antigen protein and a recombinant baculovirus comprising a gene encoding the influenza virus matrix protein 1.

7. The composition of claim 5, **characterized in that** the virus-like particles are prepared by infecting a host cell with the recombinant baculovirus comprising a gene encoding the *Toxoplasma gondii*-derived surface antigen protein and the recombinant baculovirus comprising a gene encoding influenza virus matrix protein 1 in a ratio of 3: 1 during co-infection.

8. The composition of claim 1, **characterized in that** a level of autoantibody of an individual infected with the *Toxoplasma gondii* and/or influenza virus is diagnosed.

9. A kit for multiplex diagnosis of *Toxoplasma gondii* and/or influenza virus, wherein the kit comprises the composition of any one of claims 1 to 8.

10. The kit of claim 9, wherein the diagnostic kit is any kit selected from the group consisting of an enzyme-linked immunosorbent assay (ELISA) kit, a radioimmunoassay (RIA) kit, a Sandwich assay kit, a Western Blot kit, an immunoblot assay kit, and an immunohistochemical staining kit.

11. A method for providing information necessary for multiplex diagnosis of infection with *Toxoplasma gondii* and/or influenza virus, the method comprising:
contacting a biological sample isolated from an individual with a composition for multiplex diagnosis of toxoplasmosis and/or influenza virus, which comprises virus-like particles comprising *Toxoplasma gondii*-derived surface antigen protein, and influenza virus matrix protein 1; and
detecting antibodies that bind to virus-like particles.

12. The method of claim 11, wherein the *Toxoplasma gondii*-derived surface antigen protein is any protein selected from the group consisting of AMA1, ROP4, and ROP18.
